# EUROPEAN PATENT SPECIFICATION

(11) **EP 2 900 306 B1**
(45) Date of publication and mention of the grant of the patent: **06.07.2016**
(21) Application number: 13779435.0
(22) Date of filing: 24.09.2013
(51) Int. Cl.: A61M 25/00

(54) **METHOD OF FORMING A VARIABLY REINFORCED ELONGATE MEDICAL DEVICE**
VERFAHREN ZUR HERSTELLUNG EINER VARIABEL VERSTÄRKTEN LÄNGLICHEN MEDIZINISCHEN VORRICHTUNG
PROCÉDÉ DE FABRICATION D'UN DISPOSITIF MÉDICAL ALLONGÉ À RENFORCEMENT VARIABLE

(30) Priority: 27.09.2012 US 201261706566 P
(43) Date of publication of application: 05.08.2015
(73) Proprietor: Stryker Corporation, Kalamazoo, MI 49002 (US); Stryker European Holdings I, LLC, Kalamazoo, MI 49002 (US)
(72) Inventor: PORTER, Stephen, Piedmont, California 94610 (US)
(74) Representative: Viering, Jentschura & Partner mbB Patent- und Rechtsanwälte
(86) International application number: PCT/US2013/061480
(87) International publication number: WO 2014/052335

(56) References cited:
- WO-A1-99/64098
- WO-A1-2004/033015
- US-A1- 2008 262 472

## Description

### Field of the Invention

The field of the invention pertains to catheters for use in medical interventional procedures, such as endovascular embolization by embolic coil placement. More particularly, the invention relates to methods for making reinforced intravascular catheters.

The invention relates to a method of forming a variably reinforced elongate medical device according to the pre-characterizing part of claim 1.

### Background

Intravascular catheters are used in a wide variety of minimally invasive medical procedures. Several types of catheters are utilized for intravascular treatment. Examples of intravascular catheters include guide catheters, angioplasty catheters, stent delivery devices, angiography catheters, neuro-catheters, and the like.

Such intravascular catheters may be used for diagnostic or therapeutic purposes. Generally, an intravascular catheter enables a physician to remotely perform a medical procedure by inserting the catheter into the vascular system of a patient at a location that is easily accessible and thereafter navigating the catheter to the desired target site. Using such procedures, virtually any target site in the patient's vascular system may be remotely accessed, including the coronary, cerebral, and peripheral vasculature.

In order to function efficiently, many intravascular catheters require a relatively stiff main body portion and soft distal tip. The stiff main body portion gives the intravascular catheter sufficient "pushability" and "torqueability" to allow it to be inserted, moved and rotated in the vasculature to position the distal end of the catheter at the desired site adjacent to a particular vessel. However, the distal portion should have sufficient flexibility so that it can track over a guidewire and be maneuvered through a tortuous path to the treatment site. In addition, a soft distal tip at the very distal end of the catheter should be used to minimize the risk of causing trauma to a blood vessel while the intravascular catheter is being moved through the vasculature to the proper position.

Typically, the intravascular catheter enters the patient's vasculature at a convenient location such as a blood vessel in the neck or near the groin. Once the distal portion of the catheter has entered the patient's vascular system, the physician may urge the distal tip forward by applying longitudinal forces to the proximal portion of the catheter. For the intravascular catheter to effectively communicate these longitudinal forces, it is desirable that the catheter have a high level of "pushability" and "kink resistance."

Frequently, the path taken by an intravascular catheter through the vascular system is tortuous, requiring the catheter to change direction frequently. It may also be necessary for the catheter to double back on itself. Physicians often apply torsional forces to the proximal portion of the catheter to aid in steering the catheter. To facilitate the steering process, it is desirable that an intravascular catheter have a relatively high level of "torquability." Furthermore, in order for the intravascular catheter to conform to a patient's tortuous vascular system, it is desirable that at least the distal portion of intravascular catheters be very flexible.

After the intravascular catheter has been navigated through the patient's vascular system with its distal end adjacent the target site, the catheter may be used for various diagnostic and/or therapeutic purposes. One useful therapeutic application of intravascular catheters is the treatment of intracranial aneurysms in the brain. An aneurysm which is likely to rupture, or one which has already ruptured, may be treated by delivering an embolic device or agent to the interior of the aneurysm. The embolic device or agent encourages the formation of a thrombus inside the aneurysm. The formation of a thrombus reduces the probability that an aneurysm will rupture. The formation of a thrombus also reduces the probability that a previously ruptured aneurysm will re-bleed. Thrombus agents which may be used include liquid thrombus agents such as cyanocrylate, and granulated thrombus agents such as polyvinyl alcohol. An additional type of thrombus agent which is frequently used is a miniature coil. Any of the thrombus agents described above may be delivered using an intravascular catheter.

When treating an aneurysm with the aid of an intravascular catheter, the catheter tip is typically positioned proximate the aneurysm site. The thrombus agent is then urged through the lumen of the intravascular catheter and introduced into the aneurysm. Shortly after the thrombus agent is placed in the aneurysm, a thrombus forms in the aneurysm and is shortly thereafter complemented with a collagenous material which significantly lessens the potential for aneurysm rupture. It is desirable that the lumen of the catheter provides a path for delivering embolic devices to an aneurysm. To this end, it is desirable that the pathway through the catheter have a low friction surface.

As described above, it is desirable to combine a number of performance features in an intravascular catheter. It is desirable that the proximal end of an intravascular catheter have a relatively high level of "pushability," "stability," "torqueability," "stiffness" and "kink resistance." It is also desirable that the distal end of an intravascular catheter have a relatively high level of "flexibility" and "kink resistance." The need for this combination of performance features is sometimes addressed by building a catheter which has two or more discrete tubular members having different performance characteristics. For example, a relatively flexible distal section may be bonded to a relatively rigid proximal section.

Alternatively, catheters may also have two or more discrete reinforcing members having different performance characteristics. The desired performance features of the proximal end of an intravascular catheter can be effectively achieved by incorporating a braided reinforcing member into the proximal end of the catheter. The desired performance features of the distal end of an intravascular catheter can be effectively achieved by incorporating a coiled reinforcing member into the proximal end of the catheter.

When a catheter is formed from two or more discrete tubular members or reinforcing members, it is necessary to form a bond or weld between the distal end of one tubular member or reinforcing member and the proximal end of another tubular member or reinforcing member. Such bonds or welds result in sub-optimal transition regions between proximal and distal members, leading to in-homogeneities in stiffness and kinking. The bonds or welds can also result in increased catheter diameter at the bonding region.
From the International Patent Application publication WO-A-1999/064098 a catheter with composite stiffener is known including a braid having a metal and plastic strands. Further, from International Patent Application publication WO-A-2004/033015 heat treatment of a braid to modify its properties has been known.
It is an object to improve the manufacturing method of the medical device according to the pre-characterizing part of claim 1, allowing to provide different degrees of stiffness at different locations. This and other objects are achieved by the features in the characterizing part of claim 1. Advantageous embodiments are claimed in the dependent claims 2-15.

### Summary

In one embodiment, a method of making a variably reinforced elongate medical device includes braiding a reinforcing sleeve from a first strand including a first material and a second strand including a second material, where the first material is substantially modified by exposure to a selected environmental stimulus or contact with a selected substance, and the second material is not substantially modified by exposure to the selected environmental stimulus or contact with the selected substance, exposing a portion of the braided reinforcing sleeve to the selected environmental stimulus or contacting the portion of the braided reinforcing sleeve with the selected substance, to thereby substantially modify the first strand in the respective exposed or contacted portion, and removing the substantially modified first strand from the respective exposed or contacted portion of the braided reinforcing sleeve, while the second strand is retained in the respective exposed or contacted portion.

In some embodiments, the selected environmental stimulus is a thermal environmental stimulus, such as heat, and the first material has a different melting temperature than the second material. In other embodiments, the selected environmental stimulus is radiation, the first material is substantially degraded by exposure to the radiation, and the second material is substantially intact following exposure to the radiation. The radiation may be selected from laser radiation, ultraviolet radiation or X-ray radiation. In still other embodiments, the selected substance is a solvent, and the first material has a different solubility in the solvent than the second material.

In some embodiments, removing the substantially modified first strand from the respective exposed or contacted portion of the braided reinforcing sleeve increases a flexibility of the respective exposed or contacted portion. Removing the substantially modified first strand from the respective exposed or contacted portion of the braided reinforcing sleeve may produce a reinforcing coil including the second strand in the respective exposed or contacted portion.

In other embodiments, the reinforcing sleeve is braided from a first plurality of strands including the first material and a second plurality of strands including the second material, where exposing a portion of the braided reinforcing sleeve to a selected environmental stimulus or contacting the portion of the braided reinforcing sleeve with a selected substance substantially modifies the first plurality of strands in the respective exposed or contacted portion. In such embodiments, the method also includes removing the substantially modified first plurality of strands from the respective exposed or contacted portion of the braided reinforcing sleeve, while the second plurality of strands is retained in the respective exposed or contacted portion. Removing the substantially modified first plurality of strands from the respective exposed or contacted portion of the braided reinforcing sleeve produces a multifilar reinforcing coil including the second plurality of strands in the respective exposed or contacted portion.

In another embodiment, braiding the reinforcing sleeve includes braiding a first plurality of strands with a second plurality of strands, where the first and second pluralities of strands each includes respective strands including the first material and respective strands including the second material. In such embodiments, exposing the portion of the braided reinforcing sleeve to the selected environmental stimulus or contacting the portion of the braided reinforcing sleeve with the selected substance substantially modifies the strands including the first material in the respective exposed or contacted portion. The method also includes removing the substantially modified strands including the first material from the respective exposed or contacted portion of the braided reinforcing sleeve, while the strands including the second material are retained in the respective exposed or contacted portion. In such embodiments, removing the substantially modified strands including the first material from the respective exposed or contacted portion of the braided reinforcing sleeve produces a reduced density braid including the strands including the second material in the respective exposed or contacted portion.

In some embodiments, a method of making a variably reinforced elongate medical device also includes disposing an inner liner around a mandrel, where braiding the reinforced sleeve includes braiding the first strand and the second strand on the inner liner, disposing an outer flexible jacket layer over the reinforcing sleeve after removing the substantially modified first strand from the respective exposed or contacted portion of the braided reinforcing sleeve, and laminating together the inner liner, reinforcing sleeve and outer flexible jacket layer.

In some embodiments, the first material is a polymer and the second material is a metal. The first material may be polyethylene terephthalate, nylons, aramides, aromatic polyesters or UHMWPE, and the second material may be stainless steel, nitinol, tantalum or platinum. The respective exposed or contacted portion of the braided reinforcing sleeve may be a distal portion of the reinforcing sleeve. In some embodiments, braiding the reinforcing sleeve includes winding the first strand around a mandrel in a radial direction and winding the second strand around the mandrel in an opposite radial direction.

In another embodiment, a method of forming a variably reinforced elongate medical device includes braiding a reinforcing sleeve from a first strand including a first material and a second strand including a second material, where the first material is substantially eliminated by exposure to a selected environmental stimulus or contact with a selected substance, and the second material is substantially intact following exposure to the selected environmental stimulus or contact with the selected substance, and exposing a portion of the reinforcing sleeve to the environmental stimulus or contacting the portion of the braided reinforcing sleeve with the selected substance to thereby substantially eliminate the first strand from the respective exposed or contacted portion, while the second strand is retained substantially intact in the respective exposed or contacted portion.

In some embodiments, the selected environmental stimulus is a thermal environmental stimulus, such as heat, the first material is substantially eliminated by heating, and the second material is substantially intact following heating. In other embodiments, the selected environmental stimulus is radiation, the first material is substantially eliminated by exposure to the radiation, and the second material is substantially intact following exposure to the radiation. In still other embodiments, the selected substance is a solvent, the first material is substantially eliminated by exposure to the solvent, and the second material is substantially intact following exposure to the solvent.

Other and further aspects and features of embodiments of the invention will become apparent from the ensuing detailed description in view of the accompanying figures.

### Brief Description of the Drawings

The drawings illustrate the design and utility of embodiments of the invention, in which similar elements are referred to by common reference numerals. These drawings are not necessarily drawn to scale. The relative scale of select elements may have been exaggerated for clarity. In order to better appreciate how the above-recited and other advantages and objects are obtained, a more particular description of the embodiments will be rendered, which are illustrated in the accompanying drawings. These drawings depict only typical embodiments of the invention.
FIG. 1 is a side view of a variably reinforced catheter according to an embodiment of the invention.
FIG. 2 is a detailed longitudinal cross-sectional view through the midline of a catheter according to an embodiment of the invention, at the junction between the intermediate section and the distal section.
FIGS. 3a-3f are detailed perspective views depicting a method of making a variably reinforced catheter according to an embodiment of the invention.
FIG. 4 is a flowchart summarizing the steps in a method of making a variably reinforced catheter according to an embodiment of the invention.

### Detailed Description of the Illustrated Embodiments

For terms defined in this specification, the provided definitions shall be applied throughout the specification, unless a different definition is given in the claims or elsewhere in this specification.

All numeric values are herein assumed to be modified by the term "about", whether or not explicitly indicated. The term "about" generally refers to a range of numbers that one would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Various embodiments of the invention are described hereinafter with reference to the figures. It should be noted that the figures are not drawn to scale and that elements of similar structures or functions are represented by like reference numerals throughout the figures. It should also be noted that the figures are only intended to facilitate the description of the embodiments. In addition, an illustrated embodiment of the invention need not have all advantages shown. An advantage described in conjunction with a particular embodiment of the invention is not necessarily limited to that embodiment and can be practiced in any other embodiments even if not so illustrated.

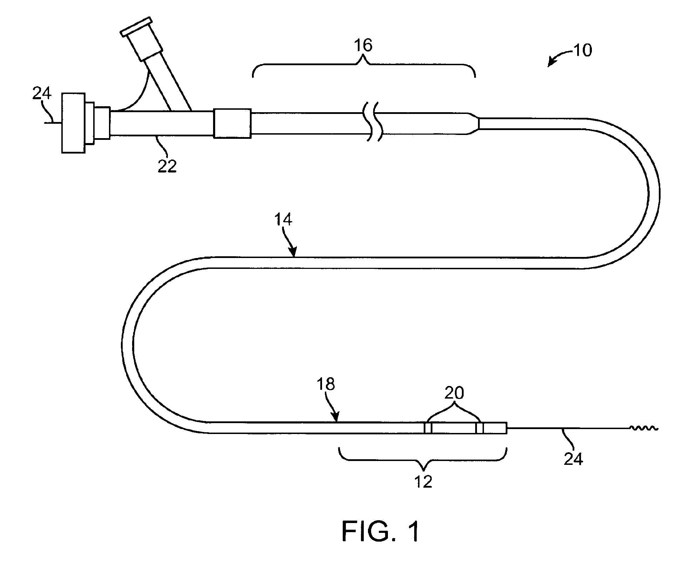

A typical neurovascular multi-section catheter 10 which may incorporate the concepts of this invention is depicted in FIG. 1. The catheter design is particularly suitable for neurological and peripheral vascular applications. Clearly, then, it is also suitable for less demanding service such as might be encountered in access and treatment of the heart. One difficulty which has arisen as higher demands for length have been placed on these catheters is that the diameter of the distal section necessarily becomes smaller and smaller. This is so since the longer catheters must reach ever narrower vascular areas. This smaller diameter requires a concomitant thinning of the wall section of the more distal portions of the catheter. This results in a further tradeoff in properties. The thinner distal section walls are able to attain even higher flexibility, which is a desirable trait because of the higher level of tortuosity in distal vasculature, but those thinner walls with their concomitant lower column strength are more prone to kinking or rippling when actively pushed along the guidewire or when vaso-occlusive devices are pushed through the catheter's lumen.

The catheter 10 depicted in FIG. 1 has a distal section 12 having significant flexibility, an intermediate section 14 which is typically less flexible than the distal section 12 without having a different outer diameter, and a long proximal section 16 which in turn is least flexible. The distal section 12 is flexible and soft to allow deep penetration of the extraordinary convolutions of the neurological vasculature without trauma. Although the distal and intermediate sections 12, 14 are distinct sections, the "junction" 18 between these two sections is seamless. Various known and often necessary accessories to the catheter assembly, e.g., one or more radiopaque marker bands 20 at the distal region to allow viewing of the position of the distal region under fluoroscopy and a Luer assembly 22 for guidewire 24 and fluids access, are also shown in FIG. 1.

The typical dimensions of neurovascular catheters are: overall length - 60-200 cm; proximal section 16 - 50-150 cm; intermediate section 14 - 5-100 cm; and distal section 12 - 2-30 cm. Obviously, these dimensions are only guidelines, are not critical to this invention, and are selected as a function of the malady treated and its site within the body.

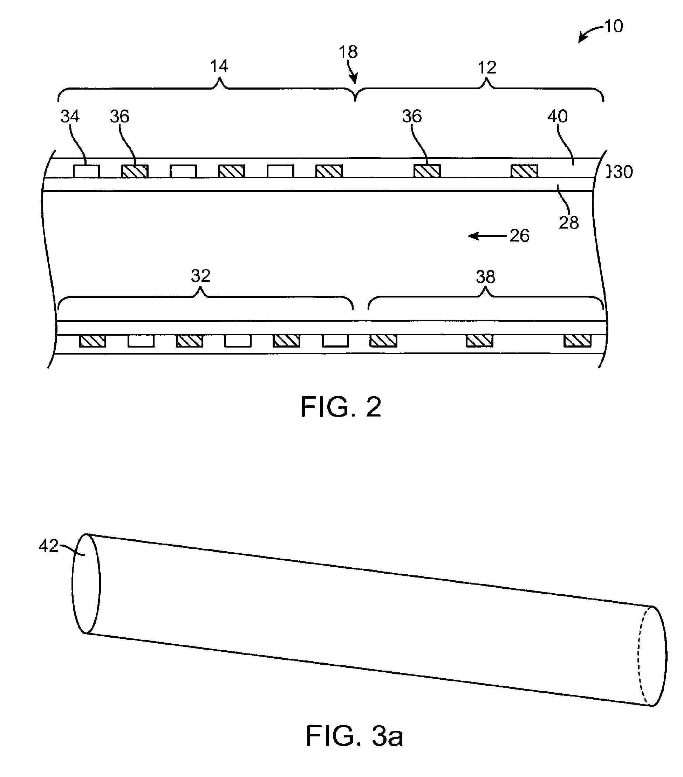

FIG 2 depicts in a longitudinal cross-sectional view a section of a catheter 10 according to an embodiment of the invention, including the junction 18 between the intermediate section 14 and the distal section 12 of the catheter 10. A lumen 26 defined by a liner 28 occupies the center of the catheter 10. The liner 28 may be made of PTFE(Teflon), FEP, polyethylene, polypropylene, polyimide, or any suitable material having a luminal surface with a low coefficient of friction. The liner 28 may be a single material layer, or it may be a double layer of two different materials.

The next layer radially outward from the liner 28 is the reinforcement layer or sleeve 30, which includes a bi-material braid 32 formed from a first strand 34 and a second strand 36 and a single-material coil 38 formed from the second strand 36. The first strand 34 is made of a first material and the second strand 36 is made of a second material. The first strand 34 is braided in a radial direction (e.g., clockwise) and the second strand 36 is braided in the opposite radial direction (e.g., counterclockwise).

While the bi-material braid 32 is depicted as being formed from two strands 34, 36, the bi-material braid 32 may be formed from more than two strands. For instance, the bi-material braid 32 may be formed from two pluralities of strands (not shown), where a first plurality of strands is made of the first material and a second plurality of strands is made of the second material. The two pluralities of strands may braided together so that each strand is individually braided, or the first plurality of strands may be braided into a first rope, which is then braided with a second rope braided from the second plurality of strands. Additional embodiments include various degrees of pre-braiding of strands (from the first, second, or a combination of the first and second pluralities) into ropes. In such embodiments, the single-material coil 38 is a multifilar coil.

Alternatively, the bi-material braid 32 may be formed from two pluralities of strands (not shown), where the first and second pluralities of strands each includes respective strands formed from the first material and respective strands formed from the second material. In such embodiments, removing the substantially modified strands including the first material from the respective exposed or contacted portion of the braided reinforcing sleeve produces a reduced density braid including the strands including the second material in the respective exposed or contacted portion.

Between the bi-material braid 32 and the single-material coil 38 is a seamless junction 18 at the location where the first strand 34 present in the bi-material braid 32 is no longer present in the single-material coil 38. The seamless junction 18 results in an even stiffness transition and reduces kinking in the catheter 10. The seamless junction 18 also allows the outer diameter of the catheter 10 to remain substantially constant from the intermediate section 14 and the distal section 12.

As will discuss below, the first material is susceptible to modification or elimination by exposure to a selected environmental stimulus or contact with a selected substance and the second material resists modification or elimination by the selected environmental stimulus or material. For instance, the first material may be polyethylene terephthalate, nylons, aramides, aromatic polyesters or UHMWPE, and the second material may be stainless steel, nitinol, tantalum or platinum.

The next layer radially outward from the reinforcement layer 30 is the flexible jacket layer 40. The flexible jacket layer 40 may be continuous throughout the proximal 16, intermediate 14 and distal 12 sections of the catheter 10. Alternatively, the flexible jacket layer 40 may include a series of thermoplastic segments that may correspond to the proximal 16, intermediate 14 and distal 12 sections. The flexible jacket layer 40 may be made of homopolymers, copolymers or polymer blends containing polyamides (e.g., nylons), polyurethanes, polyimides, polyolefins (e.g., polypropylenes, polyethylenes), fluoropolymers (e.g., FEP, PTFE, ETFE), polycarbonates, polyethers, PEEK, PVC, and other polymer resins known in the manufacture of catheter devices. The flexible jacket layer 40 may be thermoplastic or thermoset.

FIGS. 3a-3f are detailed perspective views of a section of a catheter 10 according to an embodiment of the invention, including the junction 18 between the intermediate section 14 and the distal section 12 of the catheter 10. FIGS. 3a-3f depict a method of making a variably reinforced catheter, with a focus on the formation of the junction 18 between the intermediate section 14 and the distal section 12. Proximal and distal portions of the mandrel 42 and parts of the forming catheter 10 have been omitted for clarity while focusing on the formation of the junction 18 between intermediate and distal sections 14, 12.

FIG. 3a depicts an elongate mandrel 42 around which will be formed a catheter 10. The mandrel 42 resists modification by the selected environmental stimulus that will be used in the method of making, as described below. The mandrel 42 can be made of stainless steel, nitinol, copper, and other similar materials. While the depicted mandrel 42 is cylindrical, the mandrel 42 can take on any shape according to the desired properties of the catheter 10.

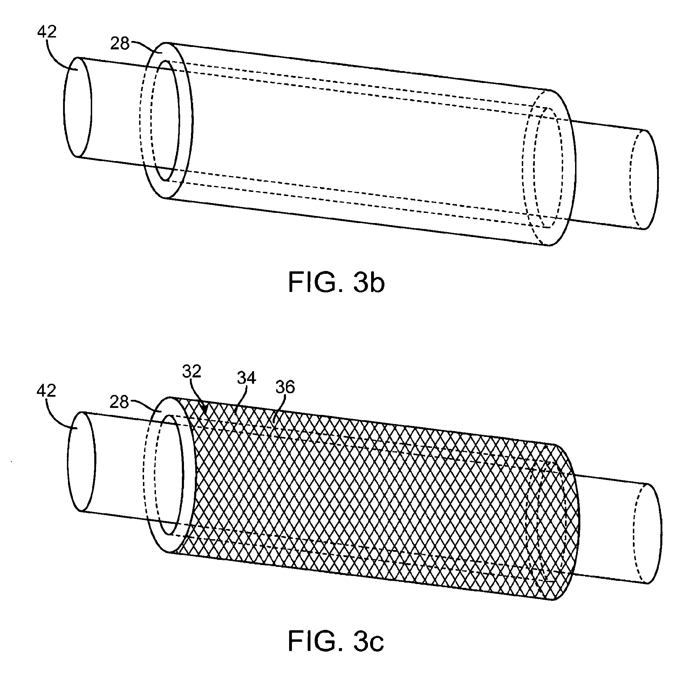

FIG. 3b depicts a liner 28, which has been disposed (e.g., stretched down onto) the mandrel 42. The liner 28 also resists modification by the selected environmental stimulus. FIG. 3c depicts a bi-material braid 32, which has been braided on the liner 28 from first strand 34 and second strand 36.

Next, a portion 44 of the bi-material braid 32 is exposed to a selected environmental stimulus or contacted with a selected substance, which either substantially modifies or eliminates the first strand 34, while leaving the second strand 36 unmodified. In some embodiments, the selected environmental stimulus is thermal, such as heat, and the first material has a lower melting temperature than the second material. Alternatively, the thermal stimulus may be cold, and the first material may be more susceptible to modification by cold than the second material.

In other embodiments, the selected environmental stimulus is radiation, such as laser radiation, ultraviolet radiation and X-ray radiation. In such embodiments, the first material is substantially degraded by exposure to the radiation, and the second material is substantially intact following exposure to the radiation. In still other embodiments, the selected substance is a solvent, and first is more soluble in the solvent than the second material.

In some embodiments, exposure or contact of the portion 44 of the bi-material braid 32 to the selected environmental stimulus or with the selected substance, modifies the first strand 34 in the exposed or contacted portion 44, rendering it more easily removed. Depending on the selected environmental stimulus or substance, the modification may include melting, degrading, partially vaporizing or dissolving. In such embodiments, the modified first strand 34 in the exposed or contacted portion 44 is removed, e.g., by washing or physically removing. In other embodiments, exposure or contact eliminates the first strand 34 in the exposed or contacted portion 44, e.g., by completely vaporizing the first strand 34.

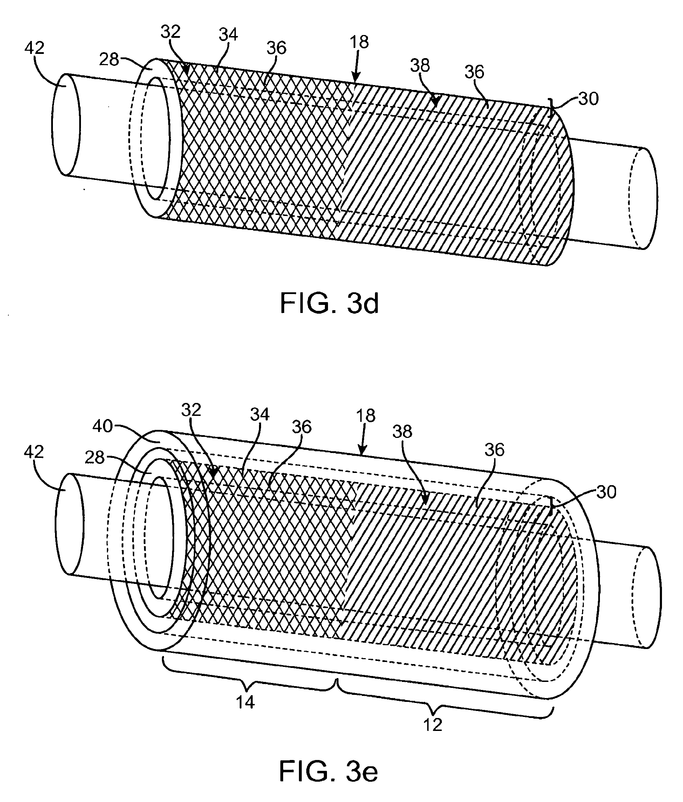

FIG. 3d depicts the forming catheter 10 section after the first strand 34 in the exposed or contacted portion 44 is removed or eliminated. As used herein, the term "remove" includes any action that renders the first strand 34 no longer part of the reinforcement layer 30. For instance, a nylon first strand 34 can be fused into a PEBAX^{®} flexible jacket layer 40 by a heat process, rendering it no longer a "reinforcing member" but a part of the flexible jacket layer 40. Because the second strand 36 in the exposed or contacted portion 44 resists modification or elimination by the selected environmental stimulus or substance, the second strand 36 remains in the exposed or contacted portion 44. The remaining second strand 36 forms a single material coil 38. The process also results in a seamless junction 18 between the bi-material braid 32 and the single material coil 38. The bi-material braid 32 and the single material coil 38 form a reinforcement layer 30 with variable degrees of reinforcement. The absence of the first strand 34 from the exposed or contacted portion 44 renders it more flexible than the portion of the catheter 10 reinforced with the bi-material braid 32.

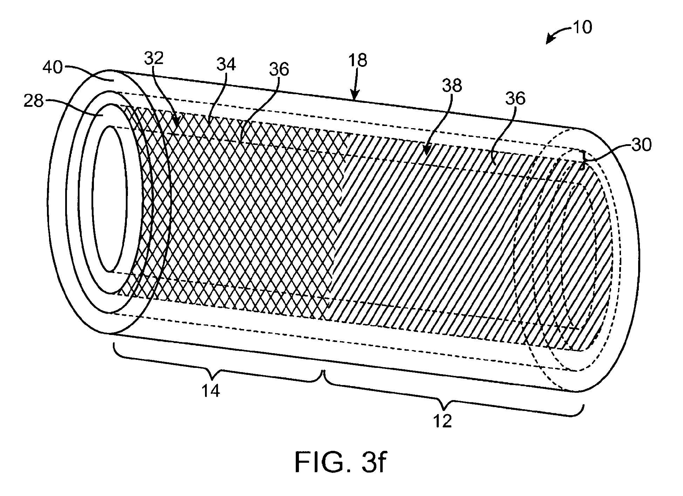

FIG. 3e depicts the next two steps in the method of making, during which a flexible jacket layer 40 is disposed over the reinforcement layer 30 and the inner liner 28, the reinforcement layer 30 and the flexible jacket layer 40 are laminated together, e.g. by heating. The lamination forms the catheter 10 from the inner liner 28, the reinforcement layer 30 and the flexible jacket layer 40, including the intermediate section 14 and the distal section 12. The flexible jacket layer 40 can be formed from polyimide, which is dip-coated and baked on over the reinforcement layer. Although only the intermediate and distal sections 14, 12 are depicted in FIG. 3e, the formed catheter 10 will have other sections, such as those depicted in FIG. 1. FIG. 3f depicts the fully formed catheter 10 section with the mandrel 42 removed.

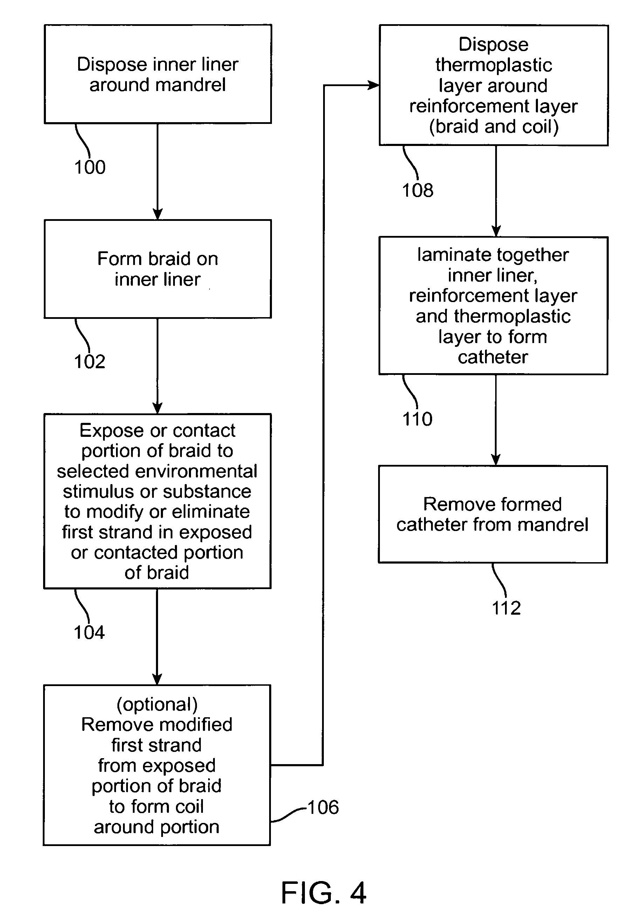

FIG. 4 is a flowchart summarizing the steps in a method of making a variably reinforced catheter according to an embodiment of the invention, consistent with the method depicted in FIGS. 3a-3f. At step 100, an inner liner 28 is disposed around a mandrel 42. At step 102, a bi-material braid 32 is braided on the inner liner 28 from first and second strands 34, 36. At step 104, a portion 44 of the bi-material braid 32, is exposed to a selected environmental stimulus or contacted with a selected substance to either modify or eliminate the first strand 34 in the exposed or contacted portion 44. At optional step 106, the modified first strand 34 in the exposed or contacted portion 44 is removed, forming a reinforcement layer 30 comprising a bi-material braid 32 and a single material coil 38. At step 108, a flexible jacket layer 40 is disposed over the reinforcement layer 30. At step 110, the reinforcement layer 30 and the flexible jacket layer 40 are laminated together to form the catheter 10. Lastly, at step 112, the fully formed catheter 10 is removed from the mandrel 42.

## Claims

1. A method of forming a variably reinforced elongate medical device (10), comprising braiding a reinforcing sleeve (30) from a first strand (34) comprising a first material and a second strand (36) comprising a second material, **characterized in that** the first material is substantially eliminated by exposure to a selected environmental stimulus or contact with a selected substance, and the second material is substantially intact following exposure to the selected environmental stimulus or contact with the selected substance; wherein a portion (44) of the reinforcing sleeve (30) is exposed to the environmental stimulus or the portion (44) of the braided reinforcing sleeve (30) is contacted with the selected substance to thereby substantially eliminate the first strand (34) from the respective exposed or contacted portion (44), while the second strand (36) is retained substantially intact in the respective exposed or contacted portion (44).

2. The method of claim 1, **characterized in that** the selected environmental stimulus is a thermal environmental stimulus.

3. The method of claim 2, **characterized in that** the thermal environmental stimulus is heat, wherein the first material is substantially eliminated by heating, and wherein the second material is substantially intact following heating.

4. The method of claim 1, **characterized in that** the selected environmental stimulus is radiation, wherein the first material is substantially eliminated by exposure to the radiation, and wherein the second material is substantially intact following exposure to the radiation.

5. The method of claim 4, **characterized in that** the radiation is selected from the group consisting of laser radiation, ultraviolet radiation and X-ray radiation.

6. The method of any of claims 1-4, **characterized in that** the selected substance is a solvent, wherein the first material is substantially eliminated by exposure to the solvent, and wherein the second material is substantially intact following exposure to the solvent.

7. The method of any of claims 1-6, **characterized by** substantially eliminating the first strand (34) from the respective exposed or contacted portion (44) of the braided reinforcing sleeve (30) increases a flexibility of the respective exposed or contacted portion (44).

8. The method of any of claims 1-6, **characterized by** substantially eliminating the first strand (34) from the respective exposed or contacted portion (44) of the braided reinforcing sleeve (30) produces a reinforcing coil (38) comprising the second strand (36) in the respective exposed or contacted portion (44).

9. The method of any of claims 1-6, **characterized in that** braiding the reinforcing sleeve (30) comprises braiding a first plurality of strands comprising the first material with a second plurality of strands comprising the second material, wherein exposing the portion (44) of the braided reinforcing sleeve (30) to the selected environmental stimulus or contacting the portion (44) of the braided reinforcing sleeve (30) with the selected substance substantially eliminates the first plurality of strands in the respective exposed or contacted portion (44), while the second plurality of strands is retained substantially intact in the respective exposed or contacted portion (44).

10. The method of claim 9, **characterized by** substantially eliminating the first plurality of strands from the respective exposed or contacted portion (44) of the braided reinforcing sleeve (30) produces a multifilar reinforcing coil (38) comprising the second plurality of strands in the respective exposed or contacted portion (44).

11. The method of any of claims 1-10, **characterized by** disposing an inner liner (28) around a mandrel (42), wherein braiding the reinforced sleeve (30) comprises braiding the first strand (34) and the second strand (36) on the inner liner (28);
disposing an outer flexible jacket layer (40) over the reinforcing sleeve (30) after substantially eliminating the first strand (34) from the respective exposed or contacted portion (44) of the braided reinforcing sleeve (30); and
laminating together the inner liner (28), reinforcing sleeve (30), and outer flexible jacket layer (40).

12. The method of any of claims 1-11, **characterized in that** the first material is a polymer and the second material is a metal.

13. The method of any of claims 1-12, **characterized in that** braiding the reinforcing sleeve comprises winding the first strand (34) around a mandrel (42) in a radial direction and winding the second strand (36) around the mandrel (42) in an opposite radial direction.

14. The method of any of claims 1-6, **characterized in that** braiding the reinforcing sleeve (30) comprises braiding a first plurality of strands with a second plurality of strands, the first and second pluralities of strands each comprising strands comprising the first material and strands comprising the second material, wherein exposing the portion (44) of the braided reinforcing sleeve (30) to the selected environmental stimulus or contacting the portion (44) of the braided reinforcing sleeve (30) with the selected substance substantially eliminates the strands comprising the first material in the respective exposed or contacted portion (44), while the strands comprising the second material are retained substantially intact in the respective exposed or contacted portion (44).

15. The method of claim 14, **characterized by** substantially eliminating the strands comprising the first material from the respective exposed or contacted portion (44) of the braided reinforcing sleeve (30) produces a reduced density braid of strands comprising the second material.

## Patentansprüche

1. Ein Verfahren zur Bildung einer variablen, verstärkten, länglichen medizinischen Vorrichtung (10), umfassend Flechten einer verstärkenden Hülle (30) aus einem ersten Strang (34), umfassend ein erstes Material, und einem zweiten Strang (36), umfassend ein zweites Material, **dadurch gekennzeichnet, dass** das erste Material im Wesentlichen dadurch entfernt wird, dass es einem ausgewählten Umweltreiz ausgesetzt wird oder mit einer ausgewählten Substanz in Kontakt kommt, und das zweite Material im Wesentlichen intakt ist, nachdem es dem ausgewählten Umweltreiz ausgesetzt wurde oder mit der ausgewählten Substanz in Kontakt kam; wobei ein Anteil (44) der verstärkenden Hülle (30) dem Umweltreiz ausgesetzt wird oder der Anteil (44) der geflochtenen, verstärkenden Hülle (30) mit der ausgewählten Substanz in Kontakt gebracht wird, um somit den ersten Strang (34) aus dem jeweiligen exponierten oder in-Kontakt-gebrachten Anteil (44) im Wesentlichen zu entfernen, während der zweite Strang (36) in dem jeweiligen exponierten oder in-Kontakt-gebrachten Anteil (44) im Wesentlichen intakt beibehalten wird.

2. Das Verfahren gemäß Anspruch 1, **gekennzeichnet dadurch, dass** der ausgewählte Umweltreiz ein thermischer Umweltreiz ist.

3. Das Verfahren gemäß Anspruch 2, **gekennzeichnet dadurch, dass** der thermische Umweltreiz Hitze ist, wobei das erste Material durch Hitze im Wesentlichen entfernt wird und wobei das zweite Material nach Hitzeeinwirkung im Wesentlichen intakt ist.

4. Das Verfahren gemäß Anspruch 1, **gekennzeichnet dadurch, dass** der ausgewählte Umweltreiz Strahlung ist, wobei das erste Material durch Strahlenexposition im Wesentlichen entfernt wird und wobei das zweite Material nach Strahlenexposition im Wesentlichen intakt ist.

5. Das Verfahren gemäß Anspruch 4, **gekennzeichnet dadurch, dass** die Strahlung ausgewählte ist aus der Gruppe bestehend aus Laserstrahlung, Ultraviolettstrahlung und Röntgenstrahlung.

6. Das Verfahren gemäß einem der Ansprüche 1-4, **gekennzeichnet dadurch, dass** die ausgewählt Substanz ein Lösemittel ist, wobei das erste Material durch Exposition zum Lösemittel im Wesentlichen entfernt wird und wobei das zweite Material nach Exposition zum Lösemittel im Wesentlichen intakt ist.

7. Das Verfahren gemäß einem der Ansprüche 1-6, **gekennzeichnet dadurch, dass** das im Wesentliche Entfernen des ersten Strangs (34) von dem jeweiligen exponierten oder in-Kontakt-gebrachten Anteil (44) der geflochtenen, verstärkenden Hülle (30) eine Flexibilität des jeweiligen exponierten oder in-Kontakt-gebrachten Anteils (44) erhöht.

8. Das Verfahren gemäß einem der Ansprüche 1-6, **gekennzeichnet dadurch, dass** das im Wesentliche Entfernen des ersten Strangs (34) von dem jeweiligen exponierten oder in-Kontakt-gebrachten Anteil (44) der geflochtenen, verstärkenden Hülle (30) eine verstärkende Windung (38) umfassend den zweiten Strang (36) in dem jeweiligen exponierten oder in-Kontakt-gebrachten Anteil (44) erzeugt.

9. Das Verfahren gemäß einem der Ansprüche 1-6, **gekennzeichnet dadurch, dass** das Flechten der verstärkenden Hülle (30) das Verflechten einer ersten Vielzahl von Strängen umfassend das erste Material mit einer zweiten Vielzahl von Strängen umfassend das zweite Material umfasst, wobei das Exponieren des Anteils (44) der geflochtenen, verstärkenden Hülle (30) gegenüber einem ausgewählten Umweltreiz oder das in-Kontakt-Bringen des Anteils (44) der geflochtenen, verstärkenden Hülle (30) mit einer ausgewählten Substanz die erste Vielzahl von Strängen in dem jeweiligen exponierten oder in-Kontakt-gebrachten Anteil (44) im Wesentlichen entfernt, während die zweite Vielzahl von Strängen in dem jeweiligen exponierten oder in-Kontakt-gebrachten Anteil (44) im Wesentlichen intakt beibehalten wird.

10. Das Verfahren gemäß Anspruch 9, **gekennzeichnet dadurch, dass** das Entfernen der ersten Vielzahl von Strängen von dem jeweiligen exponierten oder in-Kontakt-gebrachten Anteil (44) der geflochtenen, verstärkenden Hülle (30) eine multifilare verstärkende Windung (38) umfassend die zweite Vielzahl von Strängen in dem jeweiligen exponierten oder in-Kontakt-gebrachten Anteil (44) erzeugt.

11. Das Verfahren gemäß einem der Ansprüche 1-10, **gekennzeichnet durch** Anordnen eines inneren Mantels (28) um eine Spindel (42), wobei das Flechten der verstärkenden Hülle (30) das Verflechten des ersten Strangs (34) und des zweiten Strangs (36) auf dem inneren Mantel (28) umfasst;
Anordnen einer äußeren, flexiblen Ummantelungsschicht (40) über der verstärkenden Hülle (30) nach dem im Wesentlichen Entfernen des ersten Strangs (34) von dem jeweiligen exponierten oder in-Kontakt-gebrachten Anteil (44) der geflochtenen, verstärkenden Hülle (30); und
Zusammenlamellieren des inneren Mantels (28), der verstärkenden Hülle (30) und der äußeren, flexiblen Ummantelungsschicht (40).

12. Das Verfahren gemäß einem der Ansprüche 1-11, **gekennzeichnet dadurch, dass** das erste Material ein Polymer ist und das zweite Material ein Metall ist.

13. Das Verfahren gemäß einem der Ansprüche 1-12, **gekennzeichnet dadurch, dass** das Flechten der verstärkenden Hülle das Wickeln des ersten Stranges (34) um eine Spindel (42) in einer radialen Richtung und das Wickeln des zweiten Strangs (36) um die Spindel (42) in einer entgegengesetzten radialen Richtung umfasst.

14. Das Verfahren gemäß einem der Ansprüche 1-6, **gekennzeichnet dadurch, dass** das Flechten der verstärkenden Hülle (30) das Verflechten einer ersten Vielzahl von Strängen mit einer zweiten Vielzahl von Strängen umfasst, wobei die erste und die zweite Vielzahl von Strängen jeweils Stränge umfassend das erste Material und Stränge umfassend das zweite Material umfasst, wobei das Exponieren des Anteils (44) der geflochtenen, verstärkenden Hülle (30) gegenüber dem ausgewählten Umweltreiz oder das in-Kontakt-Bringen des Anteils (44) der geflochtenen, verstärkenden Hülle (30) mit der ausgewählten Substanz die Stränge umfassend das erste Material in dem jeweiligen exponierten oder in-Kontakt-gebrachten Anteil (44) im Wesentlichen entfernt, während die Stränge umfassend das zweite Material in dem jeweiligen exponierten oder in-Kontakt-gebrachten Anteil (44) im Wesentlichen beibehalten werden.

15. Das Verfahren gemäß Anspruch 14, **gekennzeichnet dadurch, dass** das im Wesentliche Entfernen der Stränge umfassend das erste Material von dem jeweiligen exponierten oder in-Kontakt-gebrachten Anteil (44) der geflochtenen, verstärkenden Hülle (30) eine verminderte Flechtdichte von Strängen umfassend das zweite Material erzeugt.

## Revendications

1. Procédé de formation d'un dispositif médical allongé à renfort variable (10), qui comprend le tressage d'un manchon de renfort (30) à partir d'un premier brin (34) qui comprend un premier matériau et d'un second brin (36) qui comprend un second matériau, **caractérisé en ce que** le premier matériau est sensiblement éliminé par exposition à un stimulus environnemental choisi ou par contact avec une substance choisie, et le second matériau est sensiblement intact après l'exposition au stimulus environnemental choisi ou le contact avec la substance choisie ; dans lequel une partie (44) du manchon de renfort (30) est exposée au stimulus environnemental ou la partie (44) du manchon de renfort tressé (30) est mise en contact avec la substance choisie afin d'éliminer sensiblement le premier brin (34) de la partie exposée ou mise en contact respective (44), tandis que le second brin (36) est maintenu sensiblement intact dans la partie exposée ou mise en contact respective (44).

2. Procédé selon la revendication 1, **caractérisé en ce que** le stimulus environnemental choisi est un stimulus environnemental thermique.

3. Procédé selon la revendication 2, **caractérisé en ce que** le stimulus environnemental thermique est de la chaleur, dans lequel le premier matériau est sensiblement éliminé par chauffage, et dans lequel le second matériau est sensiblement intact après le chauffage.

4. Procédé selon la revendication 1, **caractérisé en ce que** le stimulus environnemental choisi est un rayonnement, dans lequel le premier matériau est sensiblement éliminé par l'exposition au rayonnement, et dans lequel le second matériau est sensiblement intact après l'exposition au rayonnement.

5. Procédé selon la revendication 4, **caractérisé en ce que** le rayonnement est choisi parmi le groupe qui consiste en un rayonnement laser, un rayonnement ultraviolet et un rayonnement par rayons X.

6. Procédé selon l'une quelconque des revendications 1 à 4, **caractérisé en ce que** la substance choisie est un solvant, dans lequel le premier matériau est sensiblement éliminé par l'exposition au solvant, et dans lequel le second matériau est sensiblement intact après l'exposition au solvant.

7. Procédé selon l'une quelconque des revendications 1 à 6, **caractérisé en ce que** l'élimination substantielle du premier brin (34) de la partie exposée ou mise en contact respective (44) du manchon de renfort tressé (30) augmente la flexibilité de la partie exposée ou mise en contact respective (44).

8. Procédé selon l'une quelconque des revendications 1 à 6, **caractérisé en ce que** l'élimination substantielle du premier brin (34) de la partie exposée ou mise en contact respective (44) du manchon de renfort tressé (30) produit une bobine de renfort (38) qui comprend le second brin (36) dans la partie exposée ou mise en contact respective (44).

9. Procédé selon l'une quelconque des revendications 1 à 6, **caractérisé en ce que** le tressage du manchon de renfort (30) comprend le tressage d'une première pluralité de brins qui comprennent le premier matériau avec une seconde pluralité de brins qui comprennent le second matériau, dans lequel l'exposition de la partie (44) du manchon de renfort tressé (30) au stimulus environnemental choisi ou la mise en contact de la partie (44) du manchon de renfort tressé (30) avec la substance choisie élimine sensiblement la première pluralité de brins dans la partie exposée ou mise en contact respective (44), tandis que la seconde pluralité de brins est maintenue sensiblement intacte dans la partie exposée ou mise en contact respective (44).

10. Procédé selon la revendication 9, **caractérisé en ce que** l'élimination substantielle de la première pluralité de brins de la partie exposée ou mise en contact respective (44) du manchon de renfort tressé (30) produit une bobine de renfort multifilaire (38) qui comprend la seconde pluralité de brins dans la partie exposée ou mise en contact respective (44).

11. Procédé selon l'une quelconque des revendications 1 à 10, **caractérisé par** la disposition d'un revêtement intérieur (28) autour d'un mandrin (42), dans lequel le tressage du manchon renforcé (30) comprend le tressage du premier brin (34) et du second brin (36) sur le revêtement intérieur (28); la disposition d'une couche d'enveloppe extérieure flexible (40) par-dessus le manchon de renfort (30) après avoir sensiblement éliminé le premier brin (34) de la partie exposée ou mise en contact respective (44) du manchon de renfort tressé (30) ; et la stratification du revêtement intérieur (28), du manchon de renfort (30) et de la couche d'enveloppe extérieure flexible (40).

12. Procédé selon l'une quelconque des revendications 1 à 11, **caractérisé en ce que** le premier matériau est un polymère et le second matériau est un métal.

13. Procédé selon l'une quelconque des revendications 1 à 12, **caractérisé en ce que** le tressage du manchon de renfort comprend l'enroulement du premier brin (34) autour d'un mandrin (42) dans une direction radiale et l'enroulement du second brin (36) autour du mandrin (42) dans une direction radiale opposée.

14. Procédé selon l'une quelconque des revendications 1 à 6, **caractérisé en ce que** le tressage du manchon de renfort (30) comprend le tressage d'une première pluralité de brins avec une seconde pluralité de brins, la première et la seconde pluralités de brins comprenant chacune des brins qui comprennent le premier matériau et des brins qui comprennent le second matériau, dans lequel l'exposition de la partie (44) du manchon de renfort tressé (30) au stimulus environnemental choisi ou la mise en contact de la partie (44) du manchon de renfort tressé (30) avec la substance choisie élimine sensiblement les brins qui comprennent le premier matériau dans la partie exposée ou mise en contact respective (44), tandis que les brins qui comprennent le second matériau sont maintenus sensiblement intacts dans la partie exposée ou mise en contact respective (44).

15. Procédé selon la revendication 14, **caractérisé en ce que** l'élimination substantielle des brins qui comprennent le premier matériau de la partie exposée ou mise en contact respective (44) du manchon de renfort tressé (30) produit un tressage de brins à densité réduite qui comprennent le second matériau.
